# EUROPEAN PATENT APPLICATION

(11) **EP 1 010 801 A2**
(43) Date of publication of application: **21.06.2000**
(21) Application number: 00610027.5
(22) Date of filing: 07.03.2000
(51) Int. Cl.: D06M 23/04, B32B 5/26, D04H 1/54, D04H 1/56, A61F 13/15, D06N 3/00, D06N 7/00

(54) **Composite nonwoven materials**

(30) Priority: 19.03.1999 DK 38899
(71) Applicant: FIBERVISIONS A/S, 6800 Varde (DK)
(72) Inventor: Knud, Erik Nielsen, 6760 Ribe (DK)
(74) Representative: Plougmann, Vingtoft & Partners A/S

(57) **Abstract**

Composite nonwoven materials suitable for use as a barrier layer, e.g. a backsheet, in hygienic absorbent products such as disposable diapers, training pants, sanitary napkins, panty shields and adult incontinence products, the composite nonwovens comprising a nonwoven layer and a microporous, polymeric foam layer, wherein the polymeric foam layer has a desired degree of water-impermeability and a desired degree of air-permeability. The polymeric foam-coated, composite nonwovens are also suitable for use as a barrier layer in the building or construction industry, e.g. as an underroof.

## Description

### Field of the invention

The present invention relates to composite nonwoven materials suitable for use, e.g., in absorbent articles such as baby diapers, sanitary napkins, panty shields and adult incontinence products, and in disposable products such as medical drapes and gowns and table cloth products as well as in the building industry. Specifically, the composite nonwovens of the invention are in the form of a sandwich construction in which a nonwoven layer is provided with a polymeric foam coating with a controlled degree of liquid-impermeability and air/water vapour permeability.

### Background of the invention

Disposable hygienic products such as baby diapers typically consist of at least three types of components, namely 1 ) a liquid-permeable topsheet which is located closest to the skin of the user, 2) an absorbent core, and 3) a liquid-impermeable barrier, known as a backsheet, which forms the outer surface of the product. In addition, such products normally comprise one or more other components such as fastening tapes, elastic bands, leg cuffs, etc.

Various types of nonwoven materials are commonly used in these disposable hygienic articles. The original use of nonwovens in such articles was as a topsheet, since nonwovens are by nature porous, thereby permitting liquid to pass through the nonwoven topsheet and to be absorbed and held by the absorbent core. Later, hydrophobic, i.e. water-repellent, nonwovens were developed and used e.g. as leg cuffs in the sides of absorbent articles to prevent leakage of liquid that is in the process of being absorbed by the core.

A recent development in this field involves the use of nonwoven fabrics as part of a backsheet material. These backsheet materials (sometimes incorrectly referred to as "textile backsheets") consist of two different products which are produced separately and then laminated together: a hydrophobic nonwoven fabric and a polyolefin film. Although the nonwoven fabrics used for such backsheets are made more or less hydrophobic by means of, e.g., hydrophobic spin finishes applied to the fibres or filaments used to produce the nonwoven, they are nevertheless not sufficiently liquid-impermeable to be able to function as a liquid barrier alone. They must therefore be used together with a liquid-impermeable layer, i.e. typically a polyolefin film. These nonwoven/film laminates used in baby diapers are aimed at giving the wearer a feeling of having a textile-like outer layer, since this is perceived as being less unpleasant than an outer layer consisting only of a plastic film.

However, the lamination process is a rather costly way to produce a water-impermeable backsheet, since it involves separate production processes for the nonwoven and the film, followed by the actual lamination of the two layers to each other. It would be desirable to be able to produce backsheets with the water-impermeability of these known film-based laminated backsheets, but using a simpler and less expensive production process. Further, the prior art backsheets have the disadvantage of being impermeable to air and water vapour. As a result, absorbent articles with such backsheets can be quite uncomfortable for the used. It would therefore be a great advantage to be able to provide backsheets which in addition to being water-impermeable are permeable to air and water vapour. In addition, it would be desirable to be able to improve the comfort of such absorbent products by having a non-film-based outer layer.

Certain nonwoven materials provided with a foam coating are known for other, unrelated uses.

US 5,134,017 discloses foam-coated spunbonded or spunbonded/pointbonded nonwovens for use as protective clothing for workers exposed to dust and dirt. The uncoated basis weight of these fabrics is 0.75-2.0 oz/square yard (about 25-67 g/m²). According to claim 6 of this document, the overall weight of the coated fabrics is in the range of 1.2-2.5 oz/square yard (about 40-84 g/m²).

US 4,761,326 discloses a sterilizable, water vapour-permeable, bacterial barrier foam-coated medical fabric suitable for use as a medical instrument wrap, comprising a nonwoven material coated with a continuous, open-cell, microporous, hydrophobic foam.

### Brief disclosure of the invention

The present invention provides an alternative to the nonwoven-film laminated backsheet products discussed above by providing a nonwoven material made of e.g. synthetic staple fibres or spunbonded filaments that serves as a substrate for a polymeric foam coating, wherein the coating on the one hand allows a desired degree of water-impermeability and on the other hand allows a desired degree of permeability for air and water vapour. The result is a soft, textile-like, yet breathable composite nonwoven material, suitable for use e.g. as a backsheet in a hygienic absorbent product, with a chosen (normally high) degree of liquid-impermeability. This composite nonwoven provides a number of advantages, including a high degree of comfort for the wearer and the ability to be produced using relatively simple and inexpensive production methods.

Thus, in one aspect, the invention relates to a composite material suitable for use as a barrier layer in a hygienic absorbent product, comprising a nonwoven layer and a microporous, polymeric foam layer, wherein the polymeric foam layer provides a desired degree of water-impermeability and a desired degree of air-permeability.

In another aspect, the invention relates to a method for producing a composite nonwoven material comprising a nonwoven layer and a microporous, polymeric foam layer, the method comprising producing a web of fibres or filaments, bonding the web to produce a nonwoven layer, and applying to the nonwoven layer a polymeric foam composition to result in continuous, microporous, polymeric foam layer having a desired degree of water-impermeability and a desired degree of air-permeability.

In a further aspect, the invention relates to absorbent articles comprising a composite nonwoven material of the invention as a barrier layer.

A still further aspect of the invention relates to a composite material suitable for use as a barrier layer in the building or construction industry, comprising a nonwoven layer and a microporous, polymeric foam layer, wherein the foam layer provides the material with a desired degree of water-impermeability and a desired degree of air-permeability.

### Detailed description of the invention

As indicated above, the purpose of coating the nonwoven according to the invention is twofold, in that it imparts to the composite nonwoven 1) a substantial degree of liquid impermeability and 2) a substantial degree of air permeability. In addition to the benefits already described, the composite nonwoven is characterised by having a substantial impact resistance after having been exposed to loads, i.e. the forces to which baby diapers, sanitary napkins, panty shields, etc. are subjected under normal use, so that a sudden impact load does not result in liquid penetrating out of such an absorbent article.

In addition, the composite nonwoven materials according to the invention provide certain other important advantages compared to known materials used for e.g. backsheets in absorbent articles, including the following:
- The composite nonwovens of the invention, in which the water-impermeable polymeric foam layer has a "rubber-like" consistency, are "noise-free", as opposed to known backsheet materials which, regardless of whether they comprise a film alone or a film laminated to a nonwoven, have an unfortunate tendency to make "creaking" noises when in use due to the plastic film. This is especially undesired and, for obvious reasons, potentially embarrassing for users of feminine hygiene products and adult incontinence products.
- In the known backsheet materials comprising a laminate of a film and a nonwoven, the film is attached to the nonwoven only at the edges thereof, so that the two layers can move relative to each other. In contrast, in the composite nonwovens of the invention, the water impermeable coating layer is applied to the nonwoven layer in the form of a polymeric foam, so that the two layers are intimately attached to each other throughout their entire common surface. Thus, there is no risk of the two layers becoming separated or moving relative to one another.
- The composite nonwovens of the invention have an extremely high degree of elasticity, due to the fact that the polymeric foam coating is able to be stretched to a high degree, e.g. up to about 700%, without breaking. Due to the intimate attachment between the polymeric foam layer and the nonwoven layer, this provides the composite nonwoven as a whole with an extremely high elasticity, since the nonwoven layer benefits from the very high elasticity of the polymeric foam layer. The known laminated backsheets comprising a nonwoven and a plastic film, on the other hand, have a very low elasticity, due to the fact that the two layers are only laminated to each other at the edges.

### Nonwoven web materials

Nonwoven webs for use in the present invention may be manufactured from a wide variety of polymer materials conventionally used for the manufacture of nonwoven webs used e.g. in hygienic absorbent products, one preferred group of polymers being polyolefins. Suitable polyolefins include homo- and co-polymers of monoolefins such as ethylene, propylene, 1-butene, 4-methyl-1-pentene, etc., specific examples being isotactic or syndiotactic polypropylene, polybutylene, polyethylenes of different densities, such as high density polyethylene, low density polyethylene and linear low density polyethylene and blends of the same. Polypropylenes are particularly preferred. Instead of or in addition to a polyolefin, the nonwoven webs may be produced using other synthetic or regenerated polymers, for example polyester, polyamide, viscose/rayon, Lyocell or acetate. The nonwoven webs may be produced from a single type of fibre or filament or from a combination of two or more different fibre or filament types. For nonwoven webs produced from two or more different types of fibres or filaments, these may, e.g., comprise two or more different types of fibres or filaments produced from the polymers mentioned above or a combination of one or more types of synthetic fibres or filaments together with one or more types of natural or regenerated fibres. Suitable natural fibres include fibres of cellulose, cotton, flax, wool or hemp. Preferred fibres are pulp fibres such as CTMP (chemi-thermo-mechanical pulp), sulfite pulp or kraft pulp.

When different types of fibres or filaments are used, these may be of a different fineness and/or cross-section, according to the specific properties desired in the nonwoven.

The melts used to produce the fibres or filaments may contain various conventional fibre additives, for example calcium stearate, antioxidants, process stabilizers, and pigments, including whiteners and colourants such as TiO2, etc.

In the case of synthetic fibres or filaments, these may be either monocomponent or bicomponent fibres/filaments, the latter being for example sheath-and-core type bicomponent fibres with the core being located either eccentrically (off-center) or concentrically (substantially in the center), or of the side-by-side configuration. Hollow fibres or filaments may also be used, for example in order to reduce the specific weight of the nonwoven layer, or to reduce costs. Bicomponent fibres or filaments may, for example, have a core and sheath (or two sides) which comprise, respectively, polypropylene/polyethylene, high density polyethylene/linear low density polyethylene, polypropylene random copolymer/polyethylene, polypropylene/polypropylene random copolymer, or two other polyolefin homopolymers and/or copolymers; or polyester/polyethylene, polyester/polypropylene or polyester/copolyester.

Nonwoven webs produced in accordance with the invention may be either hydrophobic, hydrophilic or semihydrophobic/semihydrophilic, depending on the contemplated use. Often, the nonwoven webs will be produced from hydrophobic fibres or filaments or at least include a major portion of such hydrophobic fibres or filaments, for example of polypropylene, in order to promote the hydrophobicity of the composite nonwoven, thereby resulting in an optimal liquid barrier in the finished product. When the nonwoven web includes fibres or filaments which are not naturally hydrophobic, these fibres or filaments may be treated, e.g. during the spinning process, with a hydrophobic spin finish or other agent to increase their hydrophobicity. On the other hand, since the water-impermeability of the composite nonwovens of the invention is primarily determined by the coating layer, it is also possible to use relatively hydrophilic fibres or filaments, e.g. of polyester, and still obtain the benefits of liquid-impermeability together with air- and vapour-permeability. The use of such hydrophilic fibres may be preferred based on a desire to have the fibres in question in the final product, or based on an improved production economy.

For some applications, for example as medical gowns and drapes or as table cloths, the nonwovens may desirably be prepared from or include hydrophilic fibres or filaments, since for such applications it may be desirable to have one side of the coated fabric which is able to absorb a small amount of liquid. Of course, fibres or filaments that are naturally hydrophobic, such as polyolefin fibres or filaments, may if desired be treated with a suitable agent to provide them with hydrophilic properties.

For use in absorbent articles, e.g. as a backsheet, the nonwoven layer of the composite nonwoven material will generally have a basis weight of less than about 25 g/m². The basis weight of the nonwoven layer may also be somewhat lower, however, such as not more than about 22 g/m², e.g. not more than about 20 g/m². Also interesting are materials in which the nonwoven layer has a basis weight of less than about 20 g/m², such as in the range of down to about 15 g/m² or lower, e.g. down to about 10 g/m² or even lower, such as down to about 8 g/m² or lower.

One advantage of the invention is that, due to the intimate contact between the polymeric foam layer and the nonwoven layer, it gives the possibility to reduce the basis weight of the nonwoven layer to a level that would otherwise be unsuitable. In the context of the present invention, the nonwoven layer provides two basic functions, namely 1) as a substrate for applying the foam composition and 2) as a fibrous layer to provide a soft, pleasant, textile-like feeling when used as a barrier layer in hygienic absorbent product. This means that the lower limit for the basis weight of the nonwoven layer is essentially determined only by the need to fulfil these two functions. Of particular importance is the fact that once the polymeric foam layer has been applied to the nonwoven layer, the strength of the nonwoven layer itself is in many cases no longer critical as such, since the nonwoven layer benefits from the strength and elasticity of the polymeric foam layer.

This in turn makes it possible to significantly reduce the total weight of the composite nonwoven, for example a total weight of less than about 20 g/m², such as less than about 15 g/m², for example about 12-14 g/m² or perhaps even less. Of course, whether such a very low gram weight composite nonwoven material is of interest will depend on the intended use.

The nonwoven layer may, if desired, comprise two or more nonwoven sublayers. These can be prepared from the same or different types of fibres or filaments, and if desired using different production processes. The nonwoven sublayers can be attached to each other using any suitable means known in the art, for example thermobonding, adhesives, etc. As used herein, the term "nonwoven layer" is intended to encompass materials containing a single nonwoven layer as well as materials containing two or more nonwoven sublayers.

Further, it may in certain cases be desired to apply a polymeric foam layer to both sides of a nonwoven layer, for example with the aim of obtaining improved water-impermeability in a composite nonwoven for use in the building industry, or for a barrier layer with an extra high degree of water impermeability for a hygienic absorbent article.

Also of interest is the production of composite nonwovens according to the invention comprising two nonwoven layers with a polymeric foam layer between them. Such products could, for example, be produced by forming a first nonwoven layer, applying a foam composition to the first nonwoven layer, forming a second nonwoven layer on top of the foam layer, followed by appropriate treatment of the composite material by means of crushing, etc. as described below in order to obtain sufficient bonding between the foam layer and the two nonwoven layers. Such a product could, for example, be of interest for use as a combined acquisition/distribution layer and barrier layer in an absorbent article.

### Nonwoven web manufacture

Nonwoven webs for use in the present invention may be manufactured using any suitable conventional methods known in the art for producing nonwovens for e.g. the hygienic industry. Thus, the nonwovens may be produced using a drylaid technique, e.g. by carding of staple fibres, followed by bonding using e.g. thermobonding, typically by means of calender bonding or bonding in a hot-air oven, or using infrared or ultrasonic bonding. Instead of carding, webs of staple fibres may also be produced using airlaid techniques, followed by bonding using thermobonding or another bonding method.

Alternatively, nonwoven webs may be produced directly using spunlaid techniques, e.g. spunbonding, or by meltblowing. Spunlaid and meltblown webs are typically bonded using calender bonding.

As used herein, the term "fibre" includes manufactured cut lengths from filaments (i.e. staple fibres) as well as meltblown fibres and natural fibres, while a "filament" is uncut (e.g. spunbonded filaments). It will be understood that the present invention is applicable to nonwoven webs prepared from any type of fibre, filament or combination thereof.

Bonding of nonwoven webs may, where appropriate, also be performed using other methods such as hydroentanglement or needle punching, although thermobonding is generally preferred.

### Coating materials and methods

The polymeric foam coating used for the composite nonwovens of the invention is one which provides a microporous, breathable material which permits air and vapour to escape when the product is used e.g. as the backsheet of a hygienic absorbent article, but which has a desired degree of impermeability to liquids (where "liquids" include water, urine and blood). In a particular embodiment, the polymeric foam layer is substantially impermeable to liquid, so that liquid is effectively prevented from penetrating the foam layer.

As used herein, the term "substantially impermeable to liquid" or "substantially water-impermeable" refers to a polymeric foam coating which, when applied to a nonwoven layer as described herein and tested according to DIN 53 886, exhibits a water rising column (WRC) value of at least about 20 cm, preferably at least about 25 cm, more preferably at least about 30 cm, more preferably at least about 35 cm, more preferably at least about 40 cm, still more preferably at least about 50 cm.

Since the water impermeability of the polymeric foam coating is a function of factors that include the specific weight (g/m²) applied to the nonwoven, the constituents of the foam, the degree of cross-linking of the polymer matrix of the foam, and possible treatment of the foam such as crushing and thermofixation, it will be possible to vary these parameters in order to provide a given foam layer with the desired degree of water-impermeability. By way of example, it is possible to increase the water impermeability of a specific product simply by increasing the specific weight of the foam layer. Therefore, there is no real upper limit for the WRC values that can be obtained, and composite nonwovens prepared according to the invention can thus exhibit extremely high WRC values of, for example, up to 100 cm or 200 cm or even higher, while still maintaining air- and vapour-permeability. Often, however, the maximum value of the WRC value obtainable for a given product will, for reasons of economy and comfort, be dictated by the desire to use a polymeric foam layer with only the minimum specific weight necessary to obtain sufficient water-impermeability.

In the present context, the term "air permeable" refers to a degree of microporosity that, when tested according to DIN 53 887 (old standard; the corresponding new standard is DIN EN ISO 92 37) results in a desired minimum air penetration. It will be understood that the desired air permeability will be a function of the individual material and its intended use. By way of example, a suitable air permeability as determined by DIN 53 887 (or DIN EN ISO 92 37) may be one of at least about 5 l/m²/min, for example in the range of about 10-50 l/m²/min, such as about 20-40 l/m²/min.

The term "water vapour permeable" refers to the fact that the polymeric foam layer has a desired minimum level of water vapour permeability, depending on the intended use of the composite nonwoven material. Methods and apparatuses for determining water vapour resistance of textiles and similar materials are known; see e.g. Textile Research Journal, September 1986, pp. 566-568. A method commonly used in the art for determining water vapour permeability is the Procter & Gamble method for determining the moisture vapour transmission rate (MVTR). This test method is performed at a temperature of 40°C and a relative humidity of 75%, and the result is expressed in g or kg water/m²/24 hours. Typical MVTR values suitable for hygienic absorbent products are about 1-5 kg water/m²/24 hours, e.g. about 3 kg.

The coating layer is in the form of a stable microporous foam which is preferably applied to the nonwoven layer using the "crushed foam" technique, in which a thin layer of foamed, dissolved coating material, typically in an aqueous solution, and having a low specific weight (typically about 50-300 g/l in an aqueous solution), is applied to the nonwoven layer. Application of the foam solution is typically followed by additional process steps, such as hardening/curing, network structure formation, calendering, crushing and/or heating, to result in a microporous cell structure similar to that of a sponge.

The crushed foam technique is well-known in the art and involves, after drying of the applied foam composition, a crushing or calandering process in order to compress the foam. Drying of the applied foam layer can place at ambient temperature or at an elevated temperature that is appropriate for the polymer in question. Typically, drying will take place at an elevated temperature, for example in the range of about 60-160°C, depending on factors such as the type of polymer, the nature of the other constituents in the foam, and the drying time.

The purpose of the crushing is to create a good connection between the substrate, in this case the nonwoven layer, and the foam layer. During the compression of the foam, the cell structure of the foam is maintained, even though the thickness of the foam layer can be reduced significantly, for example from about 1 mm or less to e.g. about 0.01 mm or less. It should be noted that the thickness of the foam can be reduced to any desired degree, i.e. anything from no reduction at all (i.e. no compression step) to a reduction to a small fraction of the original thickness.

The degree of crushing, and thus the reduction of thickness, will depend on the combination of pressure and temperature applied, as well as on the time which the material is subjected to compression by pressure and elevated temperature. This in turn will be determined according to the intended use of the composite nonwoven. For purposes of the present invention, the pressure will typically be in the range of from just over zero to about 75 tons per meter nonwoven crosslength, e.g. about 5-50 tons per meter crosslength, and the temperature will typically be from about ambient temperature to about 300°C.

The compression procedure may, if desired, be followed by a thermofixation step in order to improve cross-linking of the polymer matrix. A thermofixation step may also be used, however, for products that have not been subjected to compression. The fixation temperature and the duration of the exposure to fixation will depend on the composition of the coating material used, the nonwoven material used, and on the water impermeability and air/vapour permeability requirements of the particular product. Typically, the thermofixation temperature will be in the range of from about 100°C to about 150°C, but may in some cases be higher than 150°C, depending on the nature of the polymer of the foam and the nature of the materials in the nonwoven layer.

Since different polymer matrices will crosslink at different temperatures, and to a degree depending on the time they are exposed to the elevated temperature, the degree of crosslinking can be adjusted by choosing a combination to temperature and time appropriate for the polymer in question and the intended use of the composite nonwoven. For example, a pure acrylic foam will require a temperature of roughly 130°C, while a pure polyurethane foam will only require a temperature of about 100°C for crosslinking. Also, the microporous structure itself can be influenced by using an appropriate catalyst, and the degree of water impermeability can be influenced by applying e.g. fluorocarbon compounds or similar compounds to the foam. It will be understood that the appropriate treatment in each individual case will readily be able to be determined by a person skilled in the art.

The foam layers are applied to the nonwovens in the form of foam compositions having a suitable density, e.g. in many cases 150 g/l or more, but the foam density can also be significantly less, e.g. down to 100 g/l or less. In an important embodiment of the invention, the foam composition has a density of less than about 100 g/l, for example a low as about 50-60 g/l or even lower, such as down to about 40 g/l. It is contemplated that the lower values will be able to be used for nonwovens having a sufficiently high fibre/filament density, e.g. nonwovens obtained using very fine fibres or filaments (low dtex fibres or filaments). It is believed that the application of foams having a density of less than about 100 g/l to nonwovens having a basis weight of less than about 25 g/m² represents a novel aspect of the invention.

The foam compositions are typically applied via a screen, using a doctor blade to even out the pattern of the screen. Suitable coating devices are known in the art and are commercially available, e.g. from Stork B.V., the Netherlands. Coating devices and techniques in general are described e.g. in ITB Nonwovens • Industrial Textiles 1/97 by Prof. Dr. Hans-Karl Rouette at pp. 6-12 and by Peter Böttcher at pp. 14-19.

It will be understood that the foam layer may be applied to the nonwoven layer either on-line (i.e. the nonwoven layer is formed and the foam layer is then applied in a single process, using a single production line) or off-line (i.e. the nonwoven layer is formed and, typically, rolled up so that it can be stored and/or transported to a separate production line where the foam layer is applied). For reasons of economy, on-line production of the composite nonwoven using a single production line will often be preferred.

The amount of coating applied is typically in the range of about 5-120 g/m² (based on dry weight). For barrier layers in hygienic absorbent products, the dry weight of the foam coating be in the lower end of this range, i.e. typically about 5-40 g/m², e.g. about 5-30 g/m². For use in the building industry, the dry weight of the coating will typically be in the upper end of this range, i.e. typically about 35-120 g/m², e.g. about 40-100 g/m². It will be understood that the actual amount used for any given product will depend on the nature of the nonwoven layer and the foam layer as well as the intended use of the product.

This foam coating layer is highly flexible and has a soft and pleasant feel, in contrast to diaper backsheet materials of a nonwoven/film laminate or a film alone. Due to the microporous nature of the coating layer, it is substantially liquid impermeable yet at the same time open for diffusion of air and vapour, which for coated nonwovens such as diaper backsheets designed to be used close to the skin means that skin and mucous membranes can breathe through the material without compromising the liquid-impermeability. This unique combination significantly improves user comfort.

Further, these same properties make it possible for composite nonwovens prepared as described above to function as an effective barrier to liquid water without hindering passage of water vapour for other uses, in particular for the building and construction industry. An example of one such advantageous use of the foam-coated composite nonwovens of the invention is as an underroof in buildings, thereby reducing or eliminating undesired condensation which can be a serious problem in known underroof materials. This makes it possible for the underroof to function optimally so that liquid water such as rainwater is effectively prevented from penetrating, while still allowing water vapour from the building to exit through the breathable composite material. These same properties can also be advantageous when the composite material of the invention is used in floor or wall constructions, for example as a water-tight layer under a wooden floor.

The coating material will typically comprise one or more components selected from the following:
- One or more polymer matrix materials, e.g. selected from polyurethanes, polyacrylates, copolymers of acrylates and vinyl acetates, ethylene vinyl acetates, copolymers of butadiene and vinyl monomers, copolymers of isoprene and other vinyl monomers, and copolymers of vinylchloride and vinyl monomers. The polymer matrix material will typically comprise about 10-90% by weight of the dry matter content of the coating, such as about 40-80%, depending on the properties desired in the coating.
- Inorganic additive components (particles) that serve as building blocks and reinforcing elements in the foam structure. These can for example include titanium dioxide, other titanium oxides, china clay, carbonates such as calcium carbonate, starch and talc. These additive components typically comprise about 1-25% by weight of the dry matter content of the coating.
- Cross-linking compounds which increase the degree of cross-linkage of the matrix polymers, thereby improving the mechanical and water-impermeability properties of the coating. Suitable cross-linking compounds are, for example, amino resins such as melamine resins and urea-formaldehyde resins, glyoxal resins and isocyanates. These compounds typically comprise 0-15% by weight of the dry matter content of the coating.
- Surface active agents which are active in the formation of the foam and which contribute to the properties of the foam, e.g. stability, water-impermeability and processability. It is possible to use a wide variety of surface active agents, depending on the desired properties. Suitable surface active agents are for example stearates, amine oxides, paraffin salts, fluorine carbonates and cellulose ethers. These components typically comprise about 2-25% by weight of the dry matter content of the coating.

In addition to the surface active agents mentioned above, the coating material components applied to the surface of the nonwoven will generally be dispersed in a material that serves as a solvent during application. Typically, the solvent will be tap water or demineralized/filtered water, the amount of water or other solvent in the mixture typically being roughly half, although the amount of water or other solvent can vary greatly depending on the basic nonwoven material to be coated and the nature of the coating material components.

Depending on the desired properties of the coating, one or more of the above-mentioned inorganic components, cross-linking compounds and surface active agents can be eliminated or substituted with alternative components, and/or additional components can be added as desired. Such additional components can for example be compounds conventionally used to provide resistance to heat, fire, bacteria, fungi, ultraviolet radiation, etc.

The desired components and their relative amounts to be used in a particular foam coating composition, as well as e.g. the amount of foam composition to be applied, will readily be able to be determined by persons skilled in the art, based on factors such as the nature of the fibres or filaments in the nonwoven layer, the density of the nonwoven layer, and the intended use of the composite nonwoven.

It has been found that hygienic articles comprising coated nonwovens according to the invention are washable, so that the articles can be washed repeatedly, e.g. at least 10 times, preferably at least about 20 times, more preferably at least about 50 times, at a temperature of 60°C without any significant reduction in the properties (water-impermeability and elasticity) of the product. This provides a great potential for applications in which it would be desirable to be able to wash and re-use a product that is otherwise "disposable" (i.e. disposable in the sense of normally being thrown away after it has been used once, for example training pants).

When used in a diaper or other absorbent product, the topsheet and backsheet fabrics are joined together at the edges in order to enclose the absorbent core between the topsheet and backsheet. This can be performed using different means, for example adhesives, ultrasonic bonding or thermal bonding. The components of the coated nonwoven of the invention will therefore in such cases be chosen so that the composite nonwoven is capable of being attached to a topsheet when the nonwoven is to be used as a backsheet.

The invention will be further illustrated by the following non-limiting examples.

### Example 1

A polyurethane-coated nonwoven was prepared by first preparing a thermobonded polypropylene nonwoven from carded polypropylene staple fibres. The fibres were 2.2 dtex fibres with a length of 40 mm. A nonwoven web with a basis weight of 20 g/m² was produced by calender bonding the carded fibres using a bonding temperature of 143°C. The coating composition was an anionic, aqueous polyurethane dispersion having a viscosity of 10-15 dPas and a pH of 9-10 (regulated to optimise the stability of the foam). The foam composition had a density of 100-200 g/l and was applied in a layer thickness of 0.7 mm, followed by drying for about 60 sec. at 110°C and thermofixation for about 3 min. at 130°C. The resulting coated nonwoven material had a WRC value of 880 mm (test method: DIN 53 886) and a microporosity of 31 l/m²/min. (DIN 53 887).

### Example 2

Composite nonwoven materials were prepared based on thermobonded nonwovens produced from carded polypropylene staple fibres treated with a hydrophobic spin finish (HY-DRY-T™ fibres, available from FiberVisions a/s, Denmark). The fibres had a fineness of about 2.3-2.8 dtex, and the (uncoated) nonwovens had a basis weight of about 23 g/m². Nonwoven samples were coated with an aqueous polyurethane coating composition having a density of 100-150 g/l. The amount of coating applied (dry weight, g/m²) and the WRC values determined according to DIN 53 886 were as follows:

| | | | | | | | | | |
|---|---|---|---|---|---|---|---|---|---|
| Coating (g/m²): | 32 | 43 | 28 | 27 | 27 | 26 | 32 | 25 | 25 |
| WRC (mm): | 400 | 500 | 380 | 350 | 380 | 350 | 430 | 355 | 420 |

## Claims

1. A composite nonwoven material suitable for use as a barrier layer in a hygienic absorbent product, comprising a nonwoven layer and a microporous, polymeric foam layer, wherein the polymeric foam layer provides a desired degree of water-impermeability and a desired degree of air-permeability.

2. A composite nonwoven material according to claim 1, wherein the nonwoven layer has a basis weight of less than about 25 g/m², e.g. not more than about 22 g/m², e.g. not more than about 20 g/m².

3. A composite nonwoven material according to claim 1 or 2, wherein the nonwoven layer comprises at least one carded, airlaid, spunbonded or meltblown layer, e.g. wherein the nonwoven layer comprises at least two nonwoven sublayers.

4. A composite nonwoven material according to any of the preceding claims, wherein the polymeric foam layer comprises a crushed foam, e.g. wherein the polymeric foam comprises at least one polymer matrix material selected from polyurethanes, polyacrylates, copolymers of acrylates and vinyl acetates, ethylene vinyl acetates, copolymers of butadiene and vinyl monomers, copolymers of isoprene and other vinyl monomers, and copolymers of vinylchloride and vinyl monomers.

5. A composite nonwoven material according to any of the preceding claims which when tested according to DIN 53 886 exhibits a water rising column (WRC) value of at least about 20 cm, preferably at least about 25 cm, more preferably at least about 30 cm, more preferably at least about 35 cm, more preferably at least about 40 cm, e.g. at least about 50 cm.

6. A method for producing a composite nonwoven material comprising a nonwoven layer and a microporous, polymeric foam layer, the method comprising producing a web of fibres or filaments, bonding the web to produce a nonwoven layer, and applying to the nonwoven layer a polymeric foam composition to result in continuous, microporous, polymeric foam layer having a desired degree of water-impermeability and a desired degree of air-permeability, the polymeric foam layer preferably being formed using the "crushed foam" technique.

7. A method according to claim 6, wherein the polymeric foam composition has a density of 50-300 g/l, e.g. a density of less than about 100 g/l.

8. An absorbent article comprising at least one barrier layer in the form of a composite nonwoven material according to any of claims 1-5, the absorbent article e.g. being a diaper, training pants, a sanitary napkin, a panty shield or an adult incontinence product, wherein the composite nonwoven material e.g. is a backsheet.

9. A composite nonwoven material suitable for use as a barrier layer in the building or construction industry, e.g. as an underroof, comprising a nonwoven layer and a microporous, polymeric foam layer, the polymeric foam layer having a desired degree of water-impermeability and a desired degree of air-permeability, the polymeric foam layer preferably comprising a crushed foam, the composite nonwoven material preferably exhibiting, when tested according to DIN 53 886, a water rising column (WRC) value of at least about 20 cm, preferably at least about 25 cm, more preferably at least about 30 cm, more preferably at least about 35 cm, more preferably at least about 40 cm, e.g. at least about 50 cm.

10. A barrier layer for a building, comprising a composite nonwoven material according to claim 9.
